# EUROPEAN PATENT APPLICATION

(11) **EP 3 560 516 A1**
(43) Date of publication of application: **30.10.2019**
(21) Application number: 18169693.1
(22) Date of filing: 27.04.2018
(51) Int. Cl.: A61K 45/06, A61K 31/437, A61K 31/506, A61K 31/519, A61K 31/575, A61P 35/00

(54) **COMBINATION THERAPY INCLUDING BETA-SITOSTEROL IN COMBINATION WITH AT LEAST ONE OF A BRAF INHIBITOR, A MEK INHIBITOR OR AN ERK INHIBITOR AND METHODS AND USE THEREOF**

(71) Applicant: Bergen Teknologioverføring AS, 5006 Bergen (NO)
(72) Inventor: Bjerkvig, Rolf, 5006 Bergen (NO); Thorsen, Frits, 5006 Bergen (NO); Prestegarden, Lars, 5006 Bergen (NO); Sundstrom, Terje, 5006 Bergen (NO)
(74) Representative: Gramm, Lins & Partner Patent- und Rechtsanwälte PartGmbB

(57) **Abstract**

The present invention relates in a first aspect to a pharmaceutical composition comprising beta-sitosterol or a homolog thereof in combination with a BRAF inhibitor and/or a MEK inhibitor and/or an ERK inhibitor. In particular, the present invention relates to a combination of the beta-sitosterol in combination with a BRAF inhibitor like vemurafenib, PLX4720 or dabrafenib or the MEK inhibitor trametinib. The composition is particularly useful in the treatment of cancer including metastatic cancer for example by oral or intravenous administration of both. The beta-sitosterol and the at least one of the BRAF inhibitor and/or MEK inhibitor and/or an ERK inhibitor being administered simultaneously, separately or sequentially. In a further aspect, a method of treating individuals suffering from cancer using the pharmaceutical composition according to the present invention is disclosed.

## Description

The present invention relates in a first aspect to a pharmaceutical composition comprising beta-sitosterol or a homolog thereof in combination with a BRAF inhibitor and/or a MEK inhibitor and/or an ERK inhibitor. In particular, the present invention relates to a combination of the beta-sitosterol in combination with a BRAF inhibitor like vemurafenib, PLX4720 or dabrafenib, or the MEK inhibitor trametinib. The composition is particularly useful in the treatment of cancer including metastatic cancer for example by oral or intravenous administration of both. The beta-sitosterol and the at least one of the BRAF inhibitor and/or the MEK inhibitor and/or the ERK inhibitor being administered simultaneously, separately or sequentially. In a further aspect, a method of treating individuals suffering from cancer using the pharmaceutical composition according to the present invention is disclosed.

### Prior Art

Cancer is a generic term for a large group of diseases that can affect any part of the body. Other terms used are (malignant) tumors and neoplasms. One defining feature of cancer is the rapid creation of abnormal cells that grow beyond their usual boundaries, and which can then invade adjoining parts of the body and spread to other organs. This spreading and invasion is referred to as metastases. Metastases are the major cause of death from cancer.

Cancer is a leading cause of death worldwide. The main types of cancer leading to overall cancer mortality each year are lung, stomach, colorectal, liver and breast. It is estimated that death from cancer continues to increase. Cancer may arise from one single cell. The transformation from a normal cell into a tumor cell is a multistage process typically a progression from a precancerous lesions to malignant tumors. The reason for the transformation may be due to external influence typically combined with individual genetic factors. These external influences include physical carcinogens, chemical carcinogens and biological carcinogens. The most common cancer therapy treatment modalities are surgery, chemotherapy, and radiation treatments. All of them have significant drawbacks in terms of side effects and patient discomfort.

The ultimate goal of treating cancer is to stop, control, or suppress processes that permit cancer growth and, in addition, to induce cancer cell death.

There are myriads of compounds and treatment regimens described in the literature, however, in particular with respect to metastatic cancer the success rate is not satisfactory.

In addition, treatment of cancer is costly which will increase further in the next years.

However, as noted, in particular for metastatic cancer, the treatment rates are not satisfactory and, thus, there is a demand for new cancer treatments having corrective effect and/or the ability to ameliorate cancer symptoms and improve the life-style of patients.

One class of cancer compounds useful in the treatment of subjects afflicted with cancer include compounds involved in the mitogen activated protein kinase (MAPK) pathway, namely, inhibitors of said pathway offer promise for defined subsets of patients. A main limiting factor is however the limited efficacy and durability beyond the blood brain barrier, e.g. in cases of metastases in the brain. The MAPK pathway represents a signaling pathway mediating cellular responses to growth signals. The ultimate function of this is to link receptor activity at the same membrane with modifications of cytoplasmic or nuclear targets that govern cell proliferation, differentiation and survival. Mutations in various Ras GTPases and the B-Raf kinase have been identified that they can lead to sustained and constitutive activity of the MAPK pathway. The signaling pathways include the Ras-Raf-MEK-ERK-kinase pathway. These kinases then phosphorylate and activate the intracellular protein kinase kinase MEK1 and MEK2. It is known that the MAPK pathway represents a suitable target for treatment of cancer including metastatic cancer. Various BRAF inhibitors and MEK inhibitors as well as ERK inhibitors are described in the art. However, the presence of metastases in particular brain metastases can compromise the structure and integral of the blood brain barrier hence, it is still a significant obstacle to efficient delivery of drugs including BRAF inhibitors. Further, cancer cells that have extravasated to the brain parenchyma may find protection within the microenvironment beyond the blood brain barrier, and be more prone to develop resistance due to subtherapeutic drug concentrations. There is still a prevailing need to find new therapeutic and preventive approaches that offer improved and sustained response, in particular with metastases including brain metastases. For melanoma various pharmaceutical compositions have been described including the treatment with vemurafenib representing a BRAF inhibitor when BRAF melanomas are present, however, resistance mechanisms may develop.

In recent years, it has been shown that several genetic and molecular drivers of melanoma modulate cellular metabolism in ways that are critical tumor development, metastases and drug resistance. Recently, a BRAF and MEK combination treatment received approval for the treatment of metastatic melanoma.

For example, the BRAF inhibitor dabrafenib is described in EP 2282636 B1 identifying benzenesulfonamide thiazole and oxazole compounds. The BRAF inhibitor vemurafenib is described e.g. in EP 06773861 A2. A suitable MEK inhibitor, namely, trametinib is described in EP 1761528 B1 referring to 5-amino-2,4,7-trioxo-3,4,7,8-tetrahydro-2H-pyrido[2,3-D]pyrimidine derivatives and related compounds for the treatment of cancer.

BETA-sitosterol is one of several phytosterols, namely, plantsterols, with chemical structures similar to that of cholesterol. BETA-sitosterol is widely distributed in the plant kingdom and found in vegetable oil, nuts, avocados and prepared foods, such as salad dressing. It is speculated that beta-sitosterol have a potentially reduced benign prostatic hyperplasia and blood cholesterol levels.

### Brief description of the present invention

In a first aspect of the present invention a pharmaceutical composition comprising beta-sitosterol or a homolog thereof in combination with a BRAF inhibitor and/or a MEK inhibitor and/or an ERK inhibitor is provided. Suitable BRAF inhibitors include ARQ 736, ASN003, BGB-283, CEP-32496, CCT3833, Dabrafenib, Encorafenib, GDC-0879, HM95573, LXH254, LY3009120, PLX3603, PLX4720, PLX8394, RAF265, Regorafenib, RO5126766, Sorafenib, TAK-580, Vemurafenib, XL281 and/or the MEK inhibitor is ARRY-300, AS703988, AZD8330, BI 847325, Binimetinib, Capmatinib, Cobimetinib, Crizotinib, E6201, GDC-0623, PD0325901, Pimasertib, Refametinib, RO4987655, Selumetinib, Trametinib, TAK-733, WX-554, and/or the ERK inhibitor is BVD-523, CC-90003, GDC-0994, KO-947, Lapatinib, LTT462, LY 3214996 or ONC201.

In a further aspect, the present invention provides a pharmaceutical composition comprising beta-sitosterol and vemurafenib or its analog, PLX4720.

In a further aspect, the present invention relates to the use of the pharmaceutical composition according to the present invention in the treatment of cancer. In an embodiment, the cancer is a metastatic cancer in particular metastatic melanoma.

Further, the present invention relates to a method of treating an individual suffering from cancer whereby the pharmaceutical composition according to the present invention of beta-sitosterol or a homolog thereof and at least one of BRAF inhibitor and MEK inhibitor are administered simultaneously, separately or sequentially.

In an embodiment, the treatment is a treatment of metastatic melanoma, in particular, metastatic melanoma with metastases in the brain.

### Brief description of the drawings

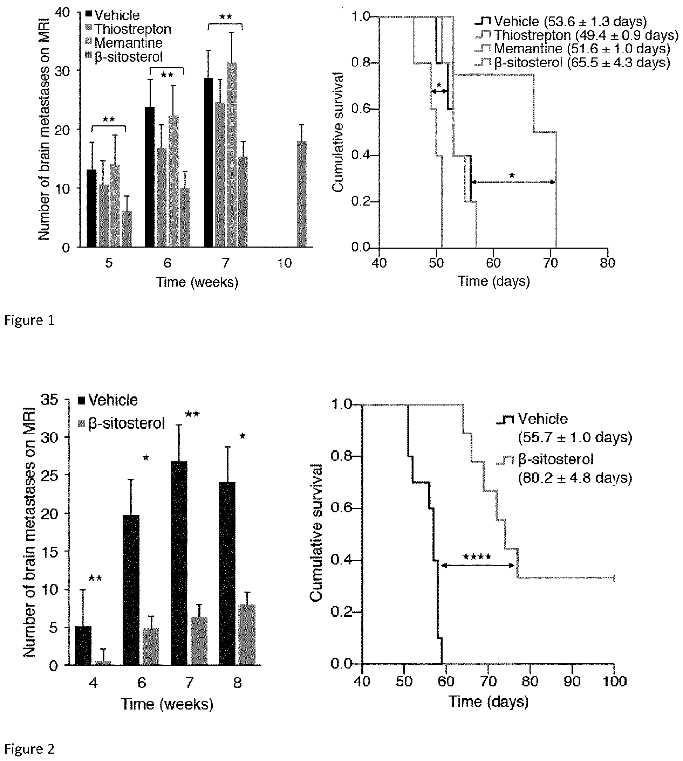

Figure 1: *In vivo* drug screening. (Figure left) Number of brain metastases at T1-weighted MRI with contrast (Student's t-test). (Figure right) Kaplan-Meier survival plot (Mantel-Cox log-rank test). There was no significant difference between vehicle- and memantine-treated mice. * P < 0.05; ** P < 0.01. All values are given as the mean ± s.e.m.
Figure 2: *In vivo* validation of β-sitosterol treatment-1. (Figure left) Number of brain metastases assessed by T1-weighted MRI with contrast (Student's t-test). The mean number of brain metastases in the vehicle group decreased slightly from seven to eight weeks as four mice with the greatest number of brain metastases were sacrificed between these observation points. (Figure right) Kaplan-Meier survival plot (Mantel-Cox log-rank test). The experiment was terminated at 100 days, and three mice in the β-sitosterol group were still alive and healthy. * P < 0.05; ** P < 0.01; **** P < 0.0001. All values are given as the mean ± s.e.m.

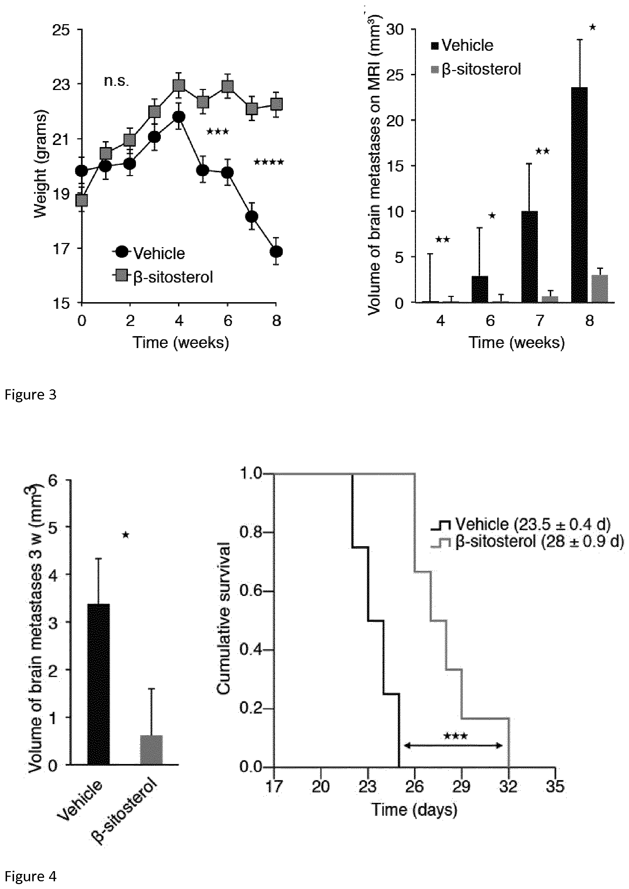

Figure 3: *In vivo* validation of β-sitosterol on H1_DL2 brain metastasis-2. (Figure left) Body weight from week zero to eight. At 100 days, the three remaining β-sitosterol mice had an average body weight of 26.7 ± 0.6 grams. (Figure right) Volume of brain metastases at MRI (T1-weighted with contrast). Total volume of brain metastases per animal was compared at group level. Student's t-test: n.s. P ≥ 0.05, * P < 0.05, ** P < 0.01, *** P < 0.001, **** P < 0.0001. All values are given as the mean ± s.e.m.
Figure 4: *In vivo* validation of β-sitosterol on PC14-PE6-Br2 brain metastases. Mice were injected intracardially with 5 x 105 PC14-PE6-Br2 cells. Test groups received daily i.p. injections of 0.1 mL vehicle (olive oil; n =4) or β-sitosterol 5 mg/kg (n = 6). (Figure left) Total volume of brain metastases at MRI at three weeks (T1-weighted with contrast). Total volume of brain metastases per animal was compared at group level. (Figure right) Kaplan-Meier survival plot (Mantel-Cox log-rank test: *** P < 0.001). All values are given as the mean ± s.e.m.

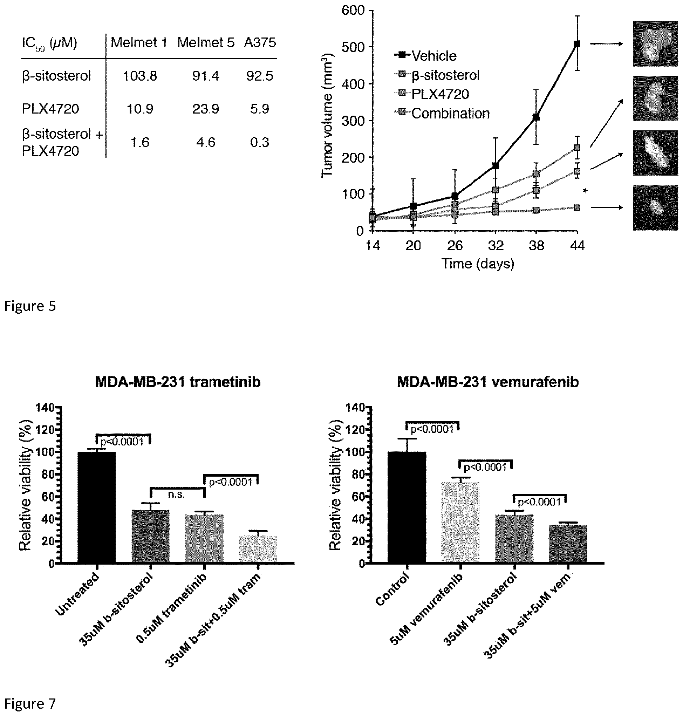

Figure 5: *In vitro* and *in vivo* effects on additional melanoma metastatic cell lines. (Figure left) Half maximal inhibitory concentration (IC50) values for β-sitosterol, PLX4720 and the combination thereof in Melmet 1, Melmet 5 and A375 cell lines (n = 3). (Figure right) Subcutaneous tumor volume ([width2 x length]/2) in mice injected with 106 Melmet 5 cells. Mice were from 2 weeks onwards given daily i.p. injections of 0.1 mL vehicle (olive oil), 20 mg/kg β-sitosterol in olive oil, 25 mg/kg PLX4720 in 0.05% DMSO, or a combination of the two latter (n = 8 in each group). Representative images of tumors at 44 days are shown in the right panel. Student's t-test: * P < 0.05, ** P < 0.01. Values are given as the mean ± s.e.m.

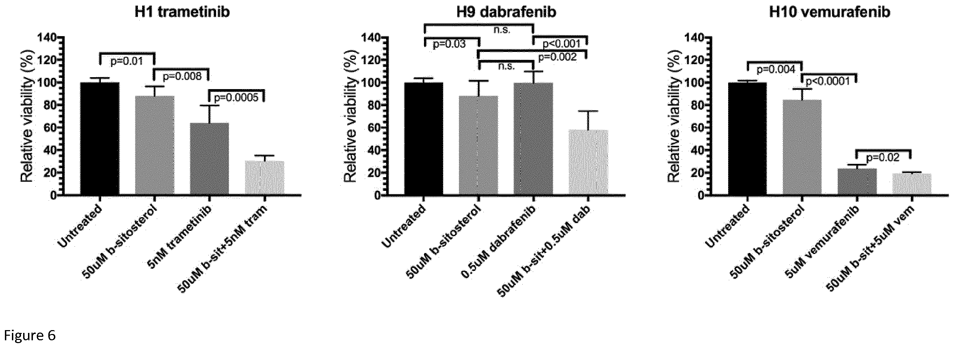

Figure 6: *In vitro* effects of other BRAF and MEK inhibitors on human melanoma brain metastatic cell lines. 5000 cells (human BRAF mutated melanoma brain metastatic cell lines H1, H9 or H10) were plated in each well of a 96 well dish. After 24 hours, the drugs were added to the wells, incubated for 72 hours, and then viability was assessed using an MTS assay. (Figure left) Combined treatment with 50uM beta-sitosterol and 5nM trametinib effectively inhibited cell viability, compared to untreated cells or single treatments. (Figure middle) Combined treatment with 50uM beta-sitosterol and 0.5uM dabrafenib effectively inhibited cell viability, compared to untreated cells or single treatments. (Figure right) Combined treatment with 50uM beta-sitosterol and 5uM vemurafenib effectively inhibited cell viability, compared to untreated cells or single treatments.
Figure 7: *In vitro* effects of BRAF and MEK inhibitors on the human breast cancer cell line MDA-MB-231. 5000 cells were plated in each well of a 96 well dish. After 24 hours, the drugs were added to the wells, incubated for 72 hours, and then viability was assessed using an MTS assay. (Figure left) Combined treatment with 35uM beta-sitosterol and 0.5uM trametinib effectively inhibited cell viability, compared to untreated cells or single treatments. (Figure right) Combined treatment with 35uM beta-sitosterol and 5uM vemurafenib effectively inhibited cell viability, compared to untreated cells or single treatments.

### Detailed description of the present invention

In a first aspect, the present invention relates to a pharmaceutical composition comprising beta-sitosterol or a homolog thereof in combination with a BRAF inhibitor and/or a MEK inhibitor and/or an ERK inhibitor, namely, in combination with at least one of a BRAF inhibitor, a MEK inhibitor and an ERK inhibitor.

In an embodiment, the beta-sitosterol or homolog thereof is beta-sitosterol.

As used herein, the term "homolog thereof", namely a beta-sitosterol homolog, refers to derivatives of beta-sitosterol maintaining the pharmaceutical activity described for beta-sitosterol but may contain chemical modifications, like different substituents in the ethyl-methyl-heptane-group at position 17. The homolog include further phytosterols on the same chemical basis, namely plantsterols, with a similar base structure to that of cholesterol. Further, the beta-sitosterol or homolog thereof may be in form of salts, hydrates or may be in form of ester. An example of a homolog is the 7-beta-hyroxy sitosterol. The skilled person is well aware of suitable homologs of beta-sitosterol accordingly. Beta-sitosterol has been recognized as being well-tolerated. Further, it represents a brain penetrable phytosterol.

The term "beta-sitosterol" is also used in form of β-sistosterol and b-sitosterol.

The present inventors surprisingly identified that the combination of a well-tolerated and brain penetrable phytosterol like beta-sitosterol with at least one of a BRAF inhibitor, an ERK inhibitor and MEK inhibitor, allows treatment of individuals suffering from cancer. In particular, metastatic cancer like melanoma with brain metastases can be treated with a combination of the beta-sitosterol or a homolog thereof with at least one of the BRAF inhibitor, ERK inhibitor and MEK inhibitor increasing survival and improve treatment.

As used herein, the term "BRAF inhibitor" refers to molecules, e.g. a chemical or biological entity in form of a prodrug or drug inhibiting the protein B-Raf. Said protein is involved in sending signals inside cells including directing cell growth. In cancer BRAF mutations are described. For example, the molecules vemurafenib and dabrafenib represent FDA approved BRAF inhibitors in particular for treating cancers driven by BRAF mutations.

The term "MEK inhibitor" as used herein refers to molecules, e.g. a chemical or biological entity in form of a prodrug or drug inhibiting the mitogen activated protein kinase kinase enzymes MEK1 and/or MEK2. Approved MEK inhibitors are trametinib and cobimetinib. For example, trametinib is FDA approved to treat BRAF mutated melanoma for example in combination with dabrafenib. Cobimetinib is an FDA approved MEK inhibitor e.g. for use in combination with vemurafenib for treatment of advanced melanoma.

The term "ERK inhibitor" as used herein refers to molecules, e.g. a chemical or biological entity in form of a prodrug or drug inhibiting the extracellular signal-regulated kinase (ERK) including ERK 1 and ERK 2.

Although the present invention is described in detail below, it is to be understood that this invention is not limited to the particular methodologies, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

In the following, the elements of the present invention will be described. These elements are listed with specific embodiments, however, it should be understood that they may be combined in any manner and in any number to create additional embodiments. The variously described examples and preferred embodiments described throughout the specification should not be construed to limit the present invention to only the explicitly described embodiments. This description should be understood to support and encompass embodiments, which combine the explicitly described embodiments with any number of the disclosed and/or preferred elements. Furthermore, any permutations and combinations of all elements described herein should be considered disclosed by the description of the present application unless the context indicates otherwise.

Throughout this specification and the claims which follow, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps although in some embodiments such other member, integer or step or group of members, integers or steps may be excluded, i.e. the subject-matter consists in the inclusion of a stated member, integer or step or group of members, integers or steps. The terms "a" and "an" and "the" and similar reference used in the context of describing the invention (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein.

All methods described herein can be performed in any suitable order unless otherwise indicated herein or otherwise clearly contradicted by context. The use of any and all examples, or exemplary language (e.g., "such as"), provided herein is intended merely to better illustrate the invention and does not pose a limitation on the scope of the invention otherwise claimed. No language in the specification should be construed as indicating any non-claimed element essential to the practice of the invention.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

When used herein "consisting of" excludes any element, step, or ingredient not specified in the claim element. When used herein, "consisting essentially of" does not exclude materials or steps that do not materially affect the basic and novel characteristics of the claim. In each instance herein any of the terms "comprising", "consisting essentially of" and "consisting of" may be replaced with either of the other two terms.

Several documents are cited throughout the text of this specification. Each of the documents cited herein (including all patents, patent applications, scientific publications, manufacturer's specifications, instructions, etc.), whether *supra* or *infra,* are hereby incorporated by reference in their entirety. Nothing herein is to be construed as an admission that the invention is not entitled to antedate such disclosure by virtue of prior invention.

In order that the present invention may be more readily understood, certain terms are first defined. Additional definitions are set forth throughout the description.

As used herein and throughout the entire description, the term "Subject" means eukaryotes, like animals, including warm blooded mammals such as humans and primates; avians; domestic household or farm animals such as cats, dogs, sheep, goats, cattle, horses and pigs; laboratory animals such as mice, rats and guinea pigs;
fish; reptiles; zoo and wild animals; and the like. The subject is preferably a mammal, more preferably a human. The term "subject", "individual" and "object" are used herein synonymously.

In an embodiment of the present invention, the BRAF inhibitor is at least one selected from i) a compound having the structure of Formula I 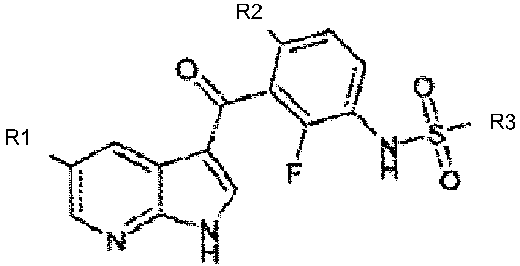 or a pharmaceutical acceptable salt, hydrate or solid thereof,
wherein R¹ is selected from the group consisting of hydrogen, halogen, optionally substituted C₁-C₆ alkyl, optionally substituted C₂-C₆ alkenyl, optionally substituted C₂-C₆ alkynyl, optionally substituted cycloalkyl, optionally substituted heterocycloalkyl, optionally substituted aryl, optionally substituted heteroaryl, -OH, -NH₂, -CN, -NO₂, - C(O)OH, -S(O)₂NH₂, -C(O)NH₂, -C(S)NH₂, -NHC(O)NH₂, -NHC(S)NH₂, - NHS(O)₂NH₂;
R² is selected from the group consisting of hydrogen, fluoro and chloro;
R³ is selected from the group consisting of optionally substituted C₂-C₆ alkyl, optionally substituted aryl, optionally substituted heteroaryl and -NR⁴R⁵;
R⁴ and R⁵ are independently hydrogen or optionally substituted C₁ - C₆ alkyl;
or R⁴ and R⁵ combine with nitrogen to which they are attached to form optionally substituted 5 - 7 membered heterocycloalkyl;
wherein optionally substituted C₁-C₆ alkyl as R¹ or C₂-C₆ alkyl as R³ are C₁-C₆ alkyl or C₂-C₆ alkyl, respectively, optionally substituted with one or more substituents selected from the group consisting of halogen like F, OH, NH₂, NO₂, CN, C(O)OH, C(S)OH, C(O)NH₂, C(S)NH₂, S(O)₂NH₂, NHC(O)NH₂, NHC(S)NH₂, NHS(O)₂NH₂, C(NH)NH₂;
wherein optionally substituted C₂-C₆ alkenyl as R¹ is C₂-C₆ alkynyl optionally substituted with one or more substituents selected from the group consisting of halogen like F, OH, NH₂, NO₂, CN, C(O)OH, C(S)OH, C(O)NH₂, C(S)NH₂, S(O)₂NH₂, NHC(O)NH₂, NHC(S)NH₂, NHS(O)₂NH₂, C(NH)NH₂;
wherein optionally substituted C₂-C₆ alkynyl as R¹ is C₂-C₆ alkynyl optionally substituted with or more substituents selected from the group consisting of halogen like F, OH, NH₂, NO₂, CN, C(O)OH, C(S)OH, C(O)NH₂, C(S)NH₂, S(O)₂NH₂, NHC(O)NH₂, NHC(S)NH₂, NHS(O)₂NH₂, C(NH)NH₂;
wherein optionally substituted cycloalkyl, optionally substituted heterocycloalkyl, optionally substituted aryl or optionally substituted heteroaryl as R¹ or R³ or optionally substituted 5 - 7 membered heterocyloalkyl as R⁴ and R⁵ combined with the nitrogen to which they are attached are cycloalkyl, heterocycloalkyl, aryl, and heteroaryl , respectively each of which is optionally substituted with one or more substituents selected from the group consisting of halogen, OH, NH₂, NO₂, CN, C(O)OH, C(S)OH, C(O)NH₂, C(S)NH₂, S(O)₂NH₂, NHC(O)NH₂, NHC(S)NH₂, NHS(O)₂NH₂, C(NH)NH₂; at each occurrence, alkenyl, by itself or as part of another substituent, is a straight or branched chain hydrocarbon having at least one carbon-to-carbon double bond; at each occurrence, alkynyl, by itself or as part of another substituent, is a straight or branched chain hydrocarbon having at least one carbon-to-carbon triple bond; at each occurrence, cycloalkyl, by itself or as part of another substituent, is a saturated or unsaturated, non-aromatic monocyclic, bicyclic or tricyclic carbon ring system of 3-10 ring members per ring; and
at each occurrence, heterocycloalkyl, by itself or as part of another substituent, is a saturated or unsaturated non-aromatic group having from 5 to 10 atoms in which from 1 to 3 carbon atoms in the ring are replaced by heteroatoms of O, S or N, and are optionally fused with benzo or heteroaryl of 5-6 ring members; or

ii) a compound of Formula II 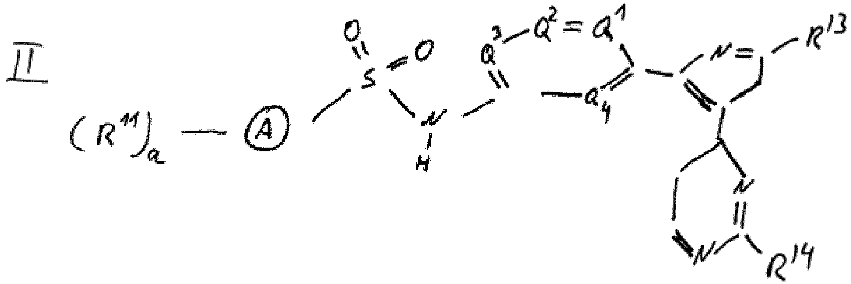 or a pharmaceutical acceptable salt, hydrate or solid thereof,
   wherein a is 0,1, 2 or 3; each R¹¹ is the same or different and is independent is selected from halo, alkyl, haloalkyl, -OR¹⁶, -CO₂R¹⁶, -NR¹⁶R¹⁷ and -CN;
   ring A is selected from C₃-C₆ cycloalkyl, phenyl 5-6 membered heterocycle and 5 - 6 membered heteroaryl, said heterocycle and said heteroaryl each having 1 or 2 heteroatoms selected from N, O and S each of Q¹, Q², Q³ and Q⁴ is CH, C-R¹² or N, wherein not more than one of Q¹, Q², Q³ and Q⁴ is N; each R¹² is the same or different is independently selected from halo, alkyl, haloalkyl, and -OR¹⁶; W is selected from -O- and -S-;
   R¹³ is selected from H, alkyl, haloalkyl-alkylene-OH and -NR⁶R⁷, C₃₋₆ cycloalkyl, alkylene, -C(O)OH, -alkylene-NH₂, and Het;
   wherein said R¹³C₃₋₆ cycloalkyl is optionally substituted with 1 or 2 substituents which are the same or different and are independently selected from halo, C₁₋₃ alkyl, haloC₁₋₃ alkyl, OH,OC₁₋₃ alkly, oxo, S(C₁₋₃ alkyl), SO₂, NH₂, N(H)C₁₋₃ alkly and N(C₁₋₃ alkyl)₂; Het is a 5 - 6 membered heterocycle having 1 or 2 heteroatoms selected from N, O and S and optionally substituted with 1 or 2 substituents which are the same or different are each independently selected from halo, C₁₋₃ alkyl, haloC₁₋₃ alkyl, OC₁₋₃ alkyl, C₁₋₃ alkylene-O-C₁₋₃ alkyl, O-C₁₋₃ alkyl, OH, C₁₋₃ alkylene-OH, oxo, SO₂(C₁₋₃ alkyl), C₁₋₃ alkylene SO₂(C₁₋₃ alkyl), NH₂, N(H)C₁₋₃ alkyl, N(C₁₋₃ alkyl)₂, CN and CH₂CN;
   R¹⁴ is selected from H, alkyl, haloalkyl, alkenyl-OR^{16,} R¹⁵-OR¹⁶, R¹⁵-CO₂, R¹⁶-, R¹⁵-SO₂R¹⁶, -R¹⁵-Het, -R¹⁵-C(O)-Het, N(H)R¹⁸, N(CH₃)R¹⁸, and -R¹⁵-NR¹⁶R¹⁷; each R¹⁵ is the same or different and is independently C₁ - C₄ alkylene, each R¹⁶ and each R¹⁷ is the same or different and is independently selected from H, alkyl, haloalkyl, -C(O) alkyl, and -C(O)-cycloalkyl;
   R¹⁸ is selected from H, alkyl (optionally substituted by -OH), haloalkyl, C₃₋₆ cycloalkyl, R¹⁵C₃₋₆ cycloalkyl, Het², R¹⁵-Het², R¹⁵OR¹⁶, R¹⁵O-R¹⁵OR¹⁶, R¹⁵-C(OH)O₂R¹⁶,
      -R¹⁵C(O)NR¹⁶R¹⁷, -R¹⁵-N(H)C(O)R¹⁶, -R⁵N(H)C(O)-R¹⁵-OR¹⁶, - R¹⁵N(H)C(O)₂-R16, -R¹⁵-NR¹⁶R¹⁷, -R¹⁵-S(O)₂R¹⁶, -R¹⁵-CN, and -R¹⁵-N(H)S(O)₂R¹⁶;
   wherein said R¹⁸C₃₋₆ clycloalkyl is optionally substituted with 1 or 2 substituents which are the same or different and independently selected from halo, C₁₋₃ alkyl, haloC₁₋₃ alkyl, OH, OC₁₋₃ alkyl, oxo, S(C₁₋₃ alkyl), SO₂(C₁₋₃ alkyl), NH₂, N(H)C₁₋₃ alkyl and N(C₁₋₃ alkyl)₂, and N(H)SO₂C₁₋₃ alkyl; and
   Het² is a 4-6 membered heterocycle having 1 or 2 heteroatoms selected from N, O and S and optionally substituted with 1, 2, 3, 4 or 5 C₁₋₃ alkyl or 1 or 2 substituents which are the same and different are independently selected from halo, C₁₋₃ alkyl, haloC₁₋₃alkyl, O-C₁₋₃alkyl, C₁₋₃alkylene-O-C₁₋₃alkyl, OH, C₁₋₃alkylene-OH, oxo, SO₂(C₁₋₃alkyl), C₁₋₃ alkylene-SO₂(C₁₋₃alkyl), NH₂, N(H)C₁₋₃alkyl, N(C₁₋₃alkyl)₂, N(H)SO₂C₁₋₃alkyl, C(O)(C₁₋₃alkyl), CO₂(C₁₋₃alkyl), CN, and -CH₂CN;
   and R¹⁹ and R²⁰ are independently selected from H and alkyl.

In a further embodiment of the invention, the MEK inhibitor is a compound of formula III or a pharmaceutical acceptable salt, hydrate or solid thereof 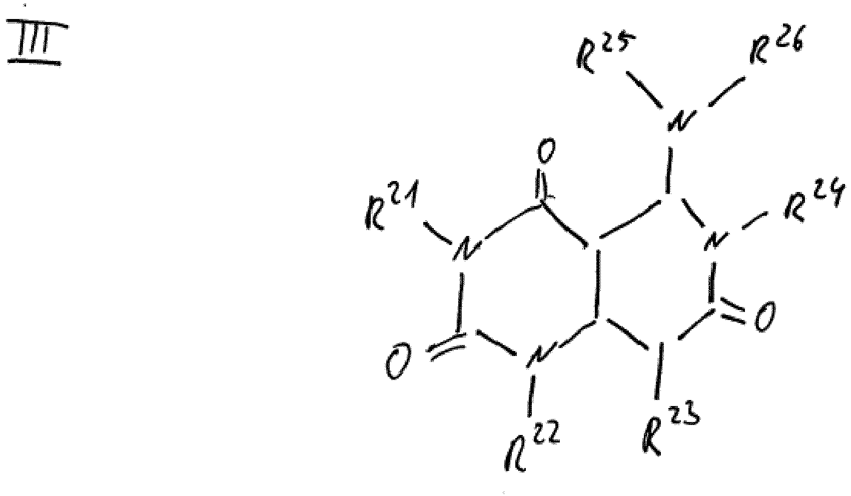
wherein R²¹, R²², and R²⁶ are the same or different and each is a C₁ - C₆ alkyl group, a C₂ - C₆ alkenyl group wherein the C₁₋₆ alkyl group and the C₂₋₆ alkenyl group are optionally substituted by 1 to 3 substituents selected from the group consisting of a halogen atom, a nitro group, a cyano group, a OR²⁷ wherein R²⁷ is a hydrogen atom or a C₁ - C₄ alkyl group, SR²⁷ wherein R²⁷ is a hydrogen atom or a C₁ - C₄ alkyl group, and NR²⁷R²⁸, -NR²⁷COR²⁸ wherein R²⁷ and R²⁸ are the same or different and each is a hydrogen atom or a C₁₋₄ alkyl group, COOR²⁷ wherein R²⁷ is a hydrogen atom or a C₁₋₄ alkyl group, in NR²⁷COR²⁸, R²⁸ is additionally a C₃ - C₁₂ carbon ring or a heterocylic group;
or - (CH₂)ₘ-Cy wherein
m is 0 or an integral of 1 to 4;
Cy is a ring of a C₃₋₁₂ carbon ring group or a heterocyclic group, wherein the heterocyclic group is a saturated or unsaturated ring group having, besides carbon atom, 1 to 4 heteroatom selected from an O, N and S, the C₃-C12 carbon ring group and the heterocyclic group are optionally substituted by 1 to 5 substituents selected from the group consisting of a halogen atom, a nitro group, a cyano group, a C₁₋₈ alkyl group, a C₂₋₄ alkenyl group, a C₂₋₄ alkynyl group, OR²⁷, SR²⁷, NR²⁷R²⁸-NR²⁷COR²⁸ wherein R²⁷ and R²⁸ are defined as above, in NR²⁷R²⁸ and in NR²⁷COR²⁸, R²⁸ is additionally a C₃ - C₁₂ carbon ring or a heterocylic group;
R²³, R²⁴ and R²⁵ are the same or different and each is a hydrogen atom, a hydroxyl-group, a C₁₋₆ alkyl group, a C₂₋₆ alkenyl group, wherein the C₁₋₆ alkyl group and the C₂₋₆ alkenyl group are optionally substituted by 1 to 3 substituents selected from the group consisting of a halogen atom, a nitro group, a cyano group, a OR²⁷ wherein R²⁷ is a hydrogen atom or a C₁ - C₄ alkyl group, SR²⁷ wherein R²⁷ is a hydrogen atom or a C₁ - C₄ alkyl group, and NR²⁷R²⁸ wherein R²⁷ and R²⁸ are the same or different and each is a hydrogen atom or a C₁₋₄ alkyl group, COOR²⁷ wherein R²⁷ is a hydrogen atom or a C₁₋₄ alkyl group; or a combination of at least two of them forming a C₁ - C₄ alkylene group.

As used herein and throughout the entire description, the term "alkyl" refers to a monoradical of a saturated straight or branched hydrocarbon. Preferably, the alkyl group comprises from 1 to 6 (such as 1 to 4) carbon atoms, i.e., 1, 2, 3, or 4, 5, or 6 carbon atoms (such as 1, 2, 3, or 4 carbon atoms). Exemplary alkyl groups include methyl, ethyl, propyl, iso-propyl, butyl, iso-butyl, tert-butyl, n-pentyl, iso-pentyl, sec-pentyl, neo-pentyl, 1,2-dimethyl-propyl, iso-amyl, n-hexyl, iso-hexyl, sec-hexyl, and the like. In some embodiments the alkyl chain is a linear. In some embodiments the alkyl chain is branched. In some embodiments the alkyl chain is substituted. In some embodiment the alkyl chain is unsubstituted. In some embodiments the alkyl chain is linear and substituted or unsubstituted. In some embodiments the alkyl chain is branched and substituted or unsubstituted.

As used herein and throughout the entire description, the term "alkenyl" refers to a monoradical of an unsaturated straight or branched hydrocarbon having at least one carbon-carbon double bond. Generally, the maximal number of carbon-carbon double bonds in the alkenyl group can be equal to the integer which is calculated by dividing the number of carbon atoms in the alkenyl group by 2 and, if the number of carbon atoms in the alkenyl group is uneven, rounding the result of the division down to the next integer. For example, for an alkenyl group having 9 carbon atoms, the maximum number of carbon-carbon double bonds is 4. Preferably, the alkenyl group has 1 to 3 carbon-carbon double bonds. Preferably, the alkenyl group comprises from 2 to 16 carbon atoms, i.e., 2, 3, 4, 5, or 6 carbon atoms, more preferably 2 to 4 carbon atoms. Thus, in a preferred embodiment, the alkenyl group comprises 2 to 6 carbon atoms and 1, 2, or 3 carbon-carbon double bonds or 2 to 4 carbon atoms and 1 or 2 carbon-carbon double bonds. The carbon-carbon double bond(s) may be in cis (Z) or trans (E) configuration. Exemplary alkenyl groups include vinyl, 1-propenyl, 2-propenyl (i.e., allyl), 1-butenyl, 2-butenyl, 3-butenyl, 1-pentenyl, 2-pentenyl, 3-pentenyl, 4-pentenyl, 1-hexenyl, 2-hexenyl, 3-hexenyl, 4-hexenyl, 5-hexenyl, and the like. If an alkenyl group is attached to a nitrogen atom, the double bond cannot be alpha to the nitrogen atom. In some embodiments the alkenyl chain is a linear. In some embodiments the alkenyl chain is branched. In some embodiments the alkenyl chain is substituted. In some embodiment the alkenyl chain is unsubstituted. In some embodiments the alkenyl chain is linear and substituted or unsubstituted. In some embodiments the alkenyl chain is branched and substituted or unsubstituted.

As used herein and throughout the entire description, the term "alkynyl" refers to a monoradical of an unsaturated straight or branched hydrocarbon having at least one carbon-carbon triple bond. Generally, the maximal number of carbon-carbon triple bonds in the alkynyl group can be equal to the integer which is calculated by dividing the number of carbon atoms in the alkynyl group by 2 and, if the number of carbon atoms in the alkynyl group is uneven, rounding the result of the division down to the next integer. For example, for an alkynyl group having 9 carbon atoms, the maximum number of carbon-carbon triple bonds is 4. The alkynyl group has 1 to 3, i.e., 1, 2, or 3, more preferably 1 or 2 carbon-carbon triple bonds. The alkynyl group comprises from 2 to 6 carbon atoms, i.e. 2, 3, 4, 5, or 6 carbon atoms or 2 to 4 carbon atoms. Thus, in a preferred embodiment, the alkynyl group comprises from 2 to 6 carbon atoms and 1, 2 or 3 carbon-carbon triple bonds or 2 to 4 carbon atoms and 1 or 2 carbon-carbon triple bonds. Exemplary alkynyl groups include ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, and the like. If an alkynyl group is attached to a nitrogen atom, the triple bond cannot be alpha to the nitrogen atom. In some embodiments the alkynyl chain is a linear. In some embodiments the alkynyl chain is branched. In some embodiments the alkynyl chain is substituted. In some embodiment the alkynyl chain is unsubstituted. In some embodiments the alkynyl chain is linear and substituted or unsubstituted. In some embodiments the alkynyl chain is branched and substituted or unsubstituted.

As used herein and throughout the entire description, the term "aryl" or "aromatic ring" refers to a monoradical of an aromatic cyclic hydrocarbon. Preferably, the aryl group contains 3 to 14 (e.g., 5 to 10, such as 5, 6, or 10) carbon atoms, more preferably 6 to 10 carbon atoms, which can be arranged in one ring (e.g., phenyl) or two or more condensed rings (e.g., naphthyl). Exemplary aryl groups include cyclopropenylium, cyclopentadienyl, phenyl, indenyl, naphthyl, azulenyl, fluorenyl, anthryl, and phenanthryl. Preferably, "aryl" refers to a monocyclic ring containing 6 carbon atoms or an aromatic bicyclic ring system containing 10 carbon atoms. Preferred examples are phenyl and naphthyl. In some embodiments the aryl is unsubstituted. In some embodiments the aryl is substituted.

As used herein and throughout the entire description, the term "heteroaryl" or "heteroaromatic ring" means an aryl group as defined above in which one or more carbon atoms in the aryl group are replaced by heteroatoms of O, S, or N. Preferably, the heteroaryl group contains 3 to 10 carbon atoms. Preferably, heteroaryl refers to a five or six-membered aromatic monocyclic ring wherein 1, 2, or 3 carbon atoms are replaced by the same or different heteroatoms of O, N, or S. Alternatively, it means an aromatic bicyclic or tricyclic ring system wherein 1, 2, 3, 4, or 5 carbon atoms are replaced with the same or different heteroatoms of O, N, or S. Preferably, in each ring of the heteroaryl group the maximum number of O atoms is 1, the maximum number of S atoms is 1, and the maximum total number of O and S atoms is 2. Exemplary heteroaryl groups include furanyl, thienyl, oxazolyl, isoxazolyl, oxadiazolyl (1,2,5- and 1,2,3-), pyrrolyl, imidazolyl, pyrazolyl, triazolyl (1,2,3- and 1,2,4-), tetrazolyl, thiazolyl, isothiazolyl, thiadiazolyl (1,2,3- and 1,2,5-), pyridyl, pyrimidinyl, pyrazinyl, triazinyl (1,2,3-, 1,2,4-, and 1,3,5-), benzofuranyl (1- and 2-), indolyl, isoindolyl, benzothienyl (1- and 2-), 1H-indazolyl, benzimidazolyl, benzoxazolyl, indoxazinyl, benzisoxazolyl, benzothiazolyl, benzisothiazolyl, benzotriazolyl, quinolinyl, isoquinolinyl, benzodiazinyl, quinoxalinyl, quinazolinyl, benzotriazinyl (1,2,3- and 1,2,4-benzotriazinyl), pyridazinyl, phenoxazinyl, thiazolopyridinyl, pyrrolothiazolyl, phenothiazinyl, isobenzofuranyl, chromenyl, xanthenyl, phenoxathiinyl, pyrrolizinyl, indolizinyl, indazolyl, purinyl, quinolizinyl, phthalazinyl, naphthyridinyl (1,5-, 1,6-, 1,7-, 1,8-, and 2,6-), cinnolinyl, pteridinyl, carbazolyl, phenanthridinyl, acridinyl, perimidinyl, phenanthrolinyl (1,7-, 1,8-, 1,10-, 3,8-, and 4,7-), phenazinyl, oxazolopyridinyl, isoxazolopyridinyl, pyrrolooxazolyl, and pyrrolopyrrolyl. Exemplary 5- or 6-memered heteroaryl groups include furanyl, thienyl, oxazolyl, isoxazolyl, oxadiazolyl (1,2,5- and 1,2,3-), pyrrolyl, imidazolyl, pyrazolyl, triazolyl (1,2,3- and 1,2,4-), thiazolyl, isothiazolyl, thiadiazolyl (1,2,3- and 1,2,5-), pyridyl, pyrimidinyl, pyrazinyl, triazinyl (1,2,3-, 1,2,4-, and 1,3,5-), and pyridazinyl. In some embodiments the heteroaryl is unsubstituted. In some embodiments the heteroaryl is substituted.
As used herein and throughout the entire description, the term "cycloalkyl" or "cycloaliphatic" represents cyclic non-aromatic versions of "alkyl" and "alkenyl" with preferably 3 to 14 carbon atoms, such as 3 to 10 carbon atoms, i.e., 3, 4, 5, 6, 7, 8, 9, or 10 carbon atoms, more preferably 3 to 8 carbon atoms, even more preferably 3 to 7 carbon atoms. Exemplary cycloalkyl groups include cyclopropyl, cyclopropenyl, cyclobutyl, cyclobutenyl, cyclopentyl, cyclopentenyl, cyclohexyl, cyclohexenyl, cycloheptyl, cycloheptenyl, cyclooctyl, cyclooctenyl, cyclononyl, cyclononenyl, cylcodecyl, cylcodecenyl, and adamantyl. The term "cycloalkyl" is also meant to include bicyclic and tricyclic versions thereof. If bicyclic rings are formed it is preferred that the respective rings are connected to each other at two adjacent carbon atoms, however, alternatively the two rings are connected via the same carbon atom, i.e., they form a spiro ring system or they form "bridged" ring systems. Preferred examples of cycloalkyl include C₃-C₈-cycloalkyl, in particular cyclopropyl, cyclobutyl, cyclopentyl, cyclohexyl, cycloheptyl, cyclooctyl, spiro[3,3]heptyl, spiro[3,4]octyl, spiro[4,3]octyl, bicyclo[4.1.0]heptyl, bicyclo[3.2.0]heptyl, bicyclo[2.2.1]heptyl, bicyclo[2.2.2]octyl, bicyclo[5.1.0]octyl, and bicyclo[4.2.0]octyl. In some embodiments the cycloalkyl is unsubstituted. In some embodiments the cycloalkyl is substituted.
As used herein and throughout the entire description, the term "heterocycloalkyl", "heterocyclyl" or "heterocyclic ring" or "heterocycle" means a cycloalkyl group as defined above in which from 1, 2, 3, or 4 carbon atoms in the cycloalkyl group are replaced by heteroatoms of O, S, or N. Preferably, in each ring of the heterocyclyl group the maximum number of O atoms is 1, the maximum number of S atoms is 1, and the maximum total number of O and S atoms is 2. The term "heterocyclyl" is also meant to encompass partially or completely hydrogenated forms (such as dihydro, tetrahydro or perhydro forms) of the above-mentioned heteroaryl groups. Exemplary heterocyclyl groups include morpholino, isochromanyl, chromanyl, pyrrolidinyl, imidazolidinyl, pyrazolidinyl, piperidinyl, piperazinyl, indolinyl, isoindolinyl, di- and tetrahydrofuranyl, di- and tetrahydrothienyl, di- and tetrahydrooxazolyl, di- and tetrahydroisoxazolyl, di- and tetrahydrooxadiazolyl (1,2,5- and 1,2,3-), dihydropyrrolyl, dihydroimidazolyl, dihydropyrazolyl, di- and tetrahydrotriazolyl (1,2,3- and 1,2,4-), di- and tetrahydrothiazolyl, di- and tetrahydrothiazolyl, di- and tetrahydrothiadiazolyl (1,2,3- and 1,2,5-), di- and tetrahydropyridyl, di- and tetrahydropyrimidinyl, di- and tetrahydropyrazinyl, di- and tetrahydrotriazinyl (1,2,3-, 1,2,4-, and 1,3,5-), di- and tetrahydrobenzofuranyl (1- and 2-), di- and tetrahydroindolyl, di- and tetrahydroisoindolyl, di- and tetrahydrobenzothienyl (1- and 2), di- and tetrahydro-1H-indazolyl, di- and tetrahydrobenzimidazolyl, di- and tetrahydrobenzoxazolyl, di- and tetrahydroindoxazinyl, di- and tetrahydrobenzisoxazolyl, di- and tetrahydrobenzothiazolyl, di- and tetrahydrobenzisothiazolyl, di- and tetrahydrobenzotriazolyl, di- and tetrahydroquinolinyl, di- and tetrahydroisoquinolinyl, di- and tetrahydrobenzodiazinyl, di- and tetrahydroquinoxalinyl, di- and tetrahydroquinazolinyl, di- and tetrahydrobenzotriazinyl (1,2,3- and 1,2,4-), di- and tetrahydropyridazinyl, di- and tetrahydrophenoxazinyl, di- and tetrahydrothiazolopyridinyl (such as 4,5,6-7-tetrahydro[1,3]thiazolo[5,4-c]pyridinyl or 4,5,6-7-tetrahydro[1,3]thiazolo[4,5-c]pyridinyl, e.g., 4,5,6-7-tetrahydro[1,3]thiazolo[5,4-c]pyridin-2-yl or 4,5,6-7-tetrahydro[1,3]thiazolo[4,5-c]pyridin-2-yl), di- and tetrahydropyrrolothiazolyl (such as 5,6-dihydro-4H-pyrrolo[3,4-d][1,3]thiazolyl), di- and tetrahydrophenothiazinyl, di- and tetrahydroisobenzofuranyl, di- and tetrahydrochromenyl, di- and tetrahydroxanthenyl, di- and tetrahydrophenoxathiinyl, di- and tetrahydropyrrolizinyl, di- and tetrahydroindolizinyl, di- and tetrahydroindazolyl, di- and tetrahydropurinyl, di- and tetrahydroquinolizinyl, di- and tetrahydrophthalazinyl, di- and tetrahydronaphthyridinyl (1,5-, 1,6-, 1,7-, 1,8-, and 2,6-), di- and tetrahydrocinnolinyl, di- and tetrahydropteridinyl, di- and tetrahydrocarbazolyl, di- and tetrahydrophenanthridinyl, di- and tetrahydroacridinyl, di- and tetrahydroperimidinyl, di- and tetrahydrophenanthrolinyl (1,7-, 1,8-, 1,10-, 3,8-, and 4,7-), di- and tetrahydrophenazinyl, di- and tetrahydrooxazolopyridinyl, di- and tetrahydroisoxazolopyridinyl, di- and tetrahydropyrrolooxazolyl, and di- and tetrahydropyrrolopyrrolyl. Exemplary 5- or 6-memered heterocyclyl groups include morpholino, pyrrolidinyl, imidazolidinyl, pyrazolidinyl, piperidinyl, piperazinyl, di- and tetrahydrofuranyl, di- and tetrahydrothienyl, di- and tetrahydrooxazolyl, di- and tetrahydroisoxazolyl, di- and tetrahydrooxadiazolyl (1,2,5- and 1,2,3-), dihydropyrrolyl, dihydroimidazolyl, dihydropyrazolyl, di- and tetrahydrotriazolyl (1,2,3- and 1,2,4-), di- and tetrahydrothiazolyl, di- and tetrahydroisothiazolyl, di- and tetrahydrothiadiazolyl (1,2,3- and 1,2,5-), di- and tetrahydropyridyl, di- and tetrahydropyrimidinyl, di- and tetrahydropyrazinyl, di- and tetrahydrotriazinyl (1,2,3-, 1,2,4-, and 1,3,5-), and di- and tetrahydropyridazinyl. In some embodiments the heterocyclyl is unsubstituted. In some embodiments the heterocyclyl is substituted.

As used herein and throughout the entire description, the term "halogen" or "halo" means fluoro, chloro, bromo, or iodo.

As used herein, the term "prodrug" refers to the IUPAC definition of said term, namely: "a compound that undergoes biotransformation before exhibiting pharmacological effects".

The present inventors aim in providing a new combination of beta-sitosterol or a homolog thereof and at least one of a BRAF inhibitor, an ERK inhibitor and a MEK inhibitor for the treatment of cancer examplified by metastatic cancer e.g. metastatic melanoma, e.g. BRAF mutant melanoma, having metastases in the brain.

In particular, a synergistic effect of beta-sitosterol with BRAF inhibitor or MEK inhibitor is demonstrated

Hence, in an embodiment, the pharmaceutical composition according to the present invention is a combination of at least beta-sitosterol and vemurafenib. In another embodiment, the pharmaceutical composition is a pharmaceutical composition comprising the beta-sitosterol or homolog thereof combined with the BRAF inhibitor of i) being vemurafenib or PLX4720.

In an embodiment of the present invention, the pharmaceutical composition according to the present invention is a pharmaceutical composition containing a combination of at least two BRAF inhibitors or a combination of at least one of a BRAF inhibitor and at least one of a MEK inhibitor. In an embodiment, the first BRAF inhibitor is a BRAF inhibitor of formula I as defined herein while the second BRAF inhibitor is a BRAF inhibitor of formula II as defined herein or the at least one MEK inhibitor is a MEK inhibitor as defined herein, e.g. of formula III and the at least one BRAF inhibitor is a BRAF inhibitor of formula I and of formula II.

In an embodiment, the pharmaceutical composition is a pharmaceutical composition comprising the beta-sitosterol or the homolog thereof in combination with at least one of the ARQ 736, ASN003, BGB-283, CEP-32496, CCT3833, Dabrafenib, Encorafenib, GDC-0879, HM95573, LXH254, LY3009120, PLX3603, PLX4720, PLX8394, RAF265, Regorafenib, RO5126766, Sorafenib, TAK-580, Vemurafenib, XL281 and/or the MEK inhibitor is ARRY-300, AS703988, AZD8330, BI 847325, Binimetinib, Capmatinib, Cobimetinib, Crizotinib, E6201, GDC-0623, PD0325901, Pimasertib, Refametinib, RO4987655, Selumetinib, Trametinib, TAK-733, WX-554, and/or the ERK inhibitor is BVD-523, CC-90003, GDC-0994, KO-947, Lapatinib, LTT462, LY 3214996 or ONC201.

In an embodiment, the BRAF inhibitor of i) is vemurafenib or PLX4720, the BRAF inhibitor of ii) is dabrafenib and the MEK inhibitor of formula III is trametinib.

The pharmaceutical composition may be a pharmaceutical composition comprising further a pharmaceutically accepted carrier, diluent or excipient.

As used herein and throughout the entire description, the terms "carrier" and "excipient" are used interchangeably herein. Pharmaceutically acceptable carriers or excipients include diluents (fillers, bulking agents, e.g. lactose, microcrystalline cellulose), disintegrants (e.g. sodium starch glycolate, croscarmellose sodium), binders (e.g. PVP, HPMC), lubricants (e.g. magnesium stearate), glidants (e.g. colloidal SiO₂), solvents/co-solvents (e.g. aqueous vehicle, Propylene glycol, glycerol), buffering agents (e.g. citrate, gluconates, lactates), preservatives (e.g. Na benzoate, parabens (Me, Pr and Bu), BKC), anti-oxidants (e.g. BHT, BHA, Ascorbic acid), wetting agents (e.g. polysorbates, sorbitan esters), anti-foaming agents (e.g. Simethicone), thickening agents (e.g. methylcellulose or hydroxyethylcellulose), sweetening agents (e.g. sorbitol, saccharin, aspartame, acesulfame), flavoring agents (e.g. peppermint, lemon oils, butter-scotch, etc), humectants (e.g. propylene, glycol, glycerol, sorbitol). The person skilled in the art will readily be able to choose suitable pharmaceutically acceptable carriers or excipients, depending, e.g., on the formulation and administration route of the pharmaceutical composition.

A non-exhaustive list of exemplary pharmaceutically acceptable carriers or excipients includes (biodegradable) liposomes; microspheres made of the biodegradable polymer poly(D,L)-lactic-coglycolic acid (PLGA), albumin microspheres; synthetic polymers (soluble); nanofibers, protein-DNA complexes; protein conjugates; erythrocytes; or virosomes. Various carrier based dosage forms comprise solid lipid nanoparticles (SLNs), polymeric nanoparticles, ceramic nanoparticles, hydrogel nanoparticles, copolymerized peptide nanoparticles, nanocrystals and nanosuspensions, nanocrystals, nanotubes and nanowires, functionalized nanocarriers, nanospheres, nanocapsules, liposomes, lipid emulsions, lipid microtubules/microcylinders, lipid microbubbles, lipospheres, lipopolyplexes, inverse lipid micelles, dendrimers, ethosomes, multicomposite ultrathin capsules, aquasomes, pharmacosomes, colloidosomes, niosomes, discomes, proniosomes, microspheres, microemulsions exosomes. Extracellular vehicles and polymeric micelles. Other suitable pharmaceutically acceptable excipients are *inter alia* described in Remington's Pharmaceutical Sciences, 15th Ed., Mack Publishing Co., New Jersey (1991) and Bauer et al., Pharmazeutische Technologie, 5th Ed., Govi-Verlag Frankfurt (1997).

The pharmaceutical composition of the invention will generally be designed for specific routes and methods of administration, for specific dosages and frequencies of administration, for specific treatments of specific diseases, with ranges of bio-availability and persistence, among other things. The materials of the composition are preferably formulated in concentrations that are acceptable for the site of administration.

Formulations and compositions thus may be designed in accordance with the invention for delivery by any suitable route of administration. In the context of the present invention, the routes of administration include:
- topical routes (such as epicutaneous, inhalational, nasal, opthalmic, auricular / aural, vaginal, mucosal) and aerosols;
- enteral routes (such as oral, gastrointestinal, sublingual, sublabial, buccal, rectal); and
- parenteral routes (such as intravenous, intraarterial, intraosseous, intramuscular, intracerebral, intracerebroventricular, epidural, intrathecal, subcutaneous, intraperitoneal, extra-amniotic, intraarticular, intracardiac, intradermal, intralesional, intrauterine, intravesical, intravitreal, transdermal, intranasal, transmucosal, intrasynovial, intraluminal).

In some embodiments the administration may be a parenteral route, in particular intravenous.

In some embodiments, the pharmaceutical composition, as disclosed herein, is administered to a subject in need thereof in an amount effective to treat said disease, like cancer. The subject is preferably a mammal. The subject is more preferably a primate, like a human subject.

As used herein and throughout the entire description, the term "amount effective" in the context of a composition or dosage form for administration to a subject refers to an amount of the composition or dosage form sufficient to provide a benefit in the treatment of the disease, to delay or minimize symptoms associated the disease, or to cure or ameliorate the disease. In particular, a therapeutically effective amount means an amount sufficient to provide a therapeutic benefit *in vivo.* Used in connection with an amount of a compound of the invention, the term preferably encompasses a non-toxic amount that improves overall therapy, reduces or avoids symptoms or causes of disease, or enhances the therapeutic efficacy of or synergies with another therapeutic agent.

Amounts effective will depend, of course, on the particular subject being treated; the severity of a condition, disease or disorder; the individual patient parameters including age, physical condition, size and weight; the duration of the treatment; the nature of concurrent therapy (if any); the specific route of administration and like factors within the knowledge and expertise of the health practitioner. These factors are well known to those of ordinary skill in the art and can be addressed with no more than routine experimentation. It is generally preferred that a maximum dose be used, that is, the highest safe dose according to sound medical judgment. It will be understood by those of ordinary skill in the art, however, that a patient may insist upon a lower dose or tolerable dose for medical reasons, psychological reasons or for virtually any other reason.

In an embodiment, the pharmaceutical compositions are adapted for oral or intravenous administration. The skilled person is well aware of suitable dosage. E.g. the dosage of the BRAF inhibitor or MEK inhibitor is in the amount as it is used in treatment of diseases so far. Beta-sitosterol may be administered in a dosage of from 0.5 gram/day to 5 gram/day, for several weeks or months.

In a further aspect, the present invention relates to the pharmaceutical composition according to the present invention for use in the treatment of cancer. As demonstrated herein, the combination of beta-sitosterol or a homolog thereof with an at least one of a BRAF inhibitor, a MEK inhibitor and ERK inhibitor allows to successfully treat cancer, in particular, stopping metastases of cancer by stop of growth of said metastases.

In an embodiment, the cancer is a metastatic cancer. In a further embodiment, the metastatic cancer is a metastatic melanoma cancer. In a further embodiment, the metastatic melanoma is a metastatic melanoma with melanomas in the brain, like BRAF-mutant melanoma with metastases in the brain.

In a further embodiment, the cancer is any one of Low grade glioma, Pediatric glioma, High grade glioma (pleomorphic xantoastrocytoma), Thyroid carcinoma (medullary, papillary, anaplastic), Oesophageal carcinoma, Meningioma, Soft tissue sarcoma, Lymphoblastic leukaemia, Hairy cell leukaemia, Non Hodgkin lymphoma, Langerhans cell histiocytosis, Multiple myeloma, Ependymoma, ATRT (atypical teratoid rhabdoid tumour), Craniopharyngioma, Neuroblastoma, Non-small cell lung, cancer (NCSLC), Malignant pleural mesothelioma, Thymic carcinoma, NET (neuroendocrine tumour), NEC (neuroendocrine carcinoma), Pheochromocytoma, Paraganglioma, Melanoma, Colorectal cancer, Seminoma, Non-seminoma, Gastrointestinal stromatumor, Gastric carcinoma, Duodenal neoplasm of gastric phenotype (DNGP), Pancreatic cancer, Small intestinal adenocarcinoma, Wilms tumour (Nephroblastoma), Renal cell carcinoma, Urothelial carcinoma, Bladder cancer (transitional cell carcinoma of the bladder), Prostate cancer, Malignant phyllodes tumor of the breasts, Biliary tract cancer, Ovarian cancer, Breast cancer (metastatic), Malignant peripheral nerve sheath tumor (MPNST), Vulvar cancer, Cutaneous squamous cell carcinoma, Squamous cell anal carcinoma, Histiocytic sarcoma, Hepatocellular carcinoma, Metanephric stromal tumour (benign), Renal cell carcinoma, Upper tract urothelial carcinoma, Gastric endocrine carcinoma (carcinoid), Adrenocortical carcinoma, Ewing sarcoma, Embryonal rhabdomyosarcoma, and Malignant phyllodes tumour. Any one of the mentioned types of cancer include metastatic forms thereof as well as treating the metastasis present e.g. in brain.

The use of the pharmaceutical composition for treatment of cancer include the administration of at least one dosage of the combination of beta-sitosterol or homolog thereof with the at least one of the BRAF inhibitor, a MEK inhibitor and an ERK inhibitor.

The pharmaceutical composition may be composed of a composition where both components of the combination are present as a mixture. Alternatively, the at least two components may be present separated from each other. The pharmaceutical composition may be in form of an intravenous infusion containing the respective inhibitor and in form of a capsule or tablet containing he beta-sitosterol component or its homolog.

In a further aspect, the present invention relates to a method of treating a subject suffering from cancer. Said method comprises the step of administering a pharmaceutical composition according to the present invention to said individual whereby the beta-sitosterol or homolog thereof and the at least one of a BRAF inhibitor, a MEK inhibitor and an ERK inhibitor are administered simultaneously, separately or sequentially.

That is, while in one aspect the administration may be effected orally or intravenously. Embodiments are encompassed wherein one of the components are administered by a first route of administration while the other component is administered through a different route of administration. Alternatively or in addition, the administration may be effected separately or sequentially which may depend on the specific combination used.

In a further embodiment, the pharmaceutical composition may be provided as a kit of part containing the two components of the beta-sitosterol or homolog thereof and the at least one of the BRAF inhibitor, the MEK inhibitor and the ERK inhibitor.

The invention will be described by way of examples further without restricting this invention thereto.

### Examples

Based on RNA sequencing of tumors in brains, adrenals, bone and ovaries from mice, we defined a 108-gene brain metastasis signature using a comparative work-flow of independent analyses of gene expression profiles from brain versus other organ metastases in a human BRAF-mutant melanoma xenograft model. The signature consisted of 54 upregulated and 54 downregulated genes. To identify potentially therapeutic compounds we queried Connectivity Map (cMap; Broad Institute) with the 108-gene brain metastasis signature. cMap revealed 1313 expression profiles with a cMap score < 0, which represented compounds with the potential to induce the opposite transcriptional response when compared with the signature. For further studies, we concentrated on the top 10 candidate anti-brain metastatic compounds. To test the efficacy of the candidate compounds, we performed monolayer viability and tumorsphere assays in four melanoma cell lines (Daphu I et al, Int J Mol Sci 2014;15:8773.8794, Netland IA et al, J Neurooncol 2016;129:57-66). IC₅₀ values were generally lower in tumorspheres than in monolayers, and there was substantial variation in cell line sensitivity. The most potent compounds for reducing viability and tumorsphere growth were thiostrepton, memantine, and β-sitosterol.

We next investigated the efficacy of thiostrepton, memantine, and β-sitosterol using a highly standardized human-to-mouse brain metastasis model (Sundstrøm T et al, Cancer Res 2013;73:2445-2456). Drug treatment was commenced one week after tumor cell injections. High-resolution magnetic resonance imaging (MRI) over the next 5-10 weeks revealed a significant reduction in the number and volume of brain metastases in β-sitosterol-treated mice when compared with vehicle-treated mice (figure 1 left). Importantly, β-sitosterol-treated mice survived significantly longer than vehicle-treated mice (figure 1 right). Thiostrepton-treated mice had a significantly shorter lifespan than those treated with vehicle, whereas memantine treatment did not affect survival.

To validate our β-sitosterol treatment findings, we carried out a new and more extensive *in vivo* study in which treatment commenced before cell injection. There were significantly fewer brain metastases in the β-sitosterol group than in the vehicle group (Figure 2 left). β-sitosterol treatment provided a significant survival benefit when compared with vehicle treatment (Figure 2 right). When the study was terminated at 100 days, three out of nine mice treated with β-sitosterol were healthy and tumor-free as evaluated by brain MRI and histology.

Vehicle-treated mice progressively lost weight from four weeks and onwards, whereas β-sitosterol-treated mice maintained a stable body weight (Figure 3 left). There were significantly lower volumes of brain metastases in the β-sitosterol group than in the vehicle group (Figure 3 right).

To assess whether or not the inhibitory effect was specific to melanoma, we also performed a comparable *in vivo* study using a brain-tropic cell line from human lung adenocarcinoma (PC14-PE6-Br2). Again, we found a significant reduction in brain metastatic burden leading to prolonged survival (Figure 4).

Next, we examined if combined treatment using β-sitosterol and a BRAF inhibitor could improve treatment efficacy. We performed monolayer viability assays in three other melanoma cell lines. Combination treatment with β-sitosterol and PLX4720 substantially reduced the IC₅₀ values for all cell lines (Figure 5 left). Acknowledging that BRAFi have limited BBB penetrability, we carried out a proof of concept study in a subcutaneous Melmet 5 melanoma model. Both PLX4720 and β-sitosterol were effective as monotherapies, but combination treatment significantly forestalled tumor growth (Figure 5 right).

Then we studied the *in vitro* effects after combining β-sitosterol with other BRAF/MEK inhibitors, on several BRAF mutated human melanoma brain metastasis cell lines. In an *in vitro* viability assay treating H1 cells with β-sitosterol and the MEK inhibitor trametinib, single treatments reduced the cell viability to around 85% (β-sitosterol) and 60% (trametinib), while combined treatment reduced cell viability to 30% (Figure 6 left). When treating H9 cells with β-sitosterol and the BRAF inhibitor dabrafenib, 0.5uM dabrafenib did not reduce cell viability, while β-sitosterol reduced the viability to 85%. However, only 55% of the cells survived after combined treatment (Figure 6 middle). H10 cells were treated with β-sitosterol and the BRAF inhibitor vemurafenib. Here, β-sitosterol reduced the cell viability to around 80% compared to the untreated cells, vemurafenib reduced the viability to around 25%, while when combining the treatments, around 20% of the cells survived (Figure 6 right).

Last, we studied the effects after combining β-sitosterol with other BRAF/MEK inhibitors, on the BRAF mutated human breast cancer cell line MDA-MB-231. When the cells were treated with β-sitosterol and the MEK inhibitor trametinib, single treatments reduced the cell viability to around 48% (β-sitosterol) and 44% (trametinib), while combined treatment reduced cell viability to 25% (Figure 7 left). After treatment with the BRAF inhibitor vemurafenib, single treatments reduced the cell viability to around 44% (β-sitosterol) and 73% (vemurafenib), while combined treatment reduced cell viability to 35% (Figure 7 right).

## Claims

1. Pharmaceutical composition comprising beta-sitosterol or a homolog thereof in combination with a BRAF inhibitor and/or a MEK inhibitor and/or an ERK inhibitor.

2. Pharmaceutical composition according to claim 1 wherein the beta-sitosterol or homolog thereof is beta-sitosterol.

3. The pharmaceutical composition according to anyone of claims 1 or 2 wherein the BRAF inhibitor is at least one selected from
i) a compound having the structure of Formula I 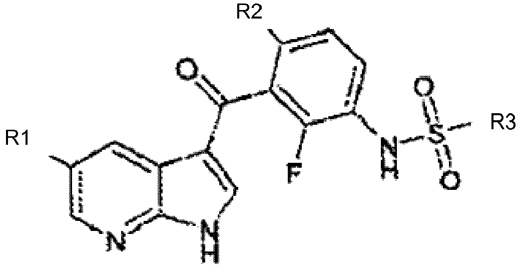
or a pharmaceutical acceptable salt, hydrate or solid thereof, wherein R¹ is selected from the group consisting of hydrogen, halogen, optionally substituted C₁-C₆ alkyl, optionally substituted C₂-C₆ alkenyl, optionally substituted C₂-C₆ alkynyl, optionally substituted cycloalkyl, optionally substituted heterocycloalkyl, optionally substituted aryl, optionally substituted heteroaryl, -OH, -NH₂, -CN, - NO₂, -C(O)OH, -S(O)₂NH₂, -C(O)NH₂, -C(S)NH₂, -NHC(O)NH₂, -NHC(S)NH₂, - NHS(O)₂NH₂;
R² is selected from the group consisting of hydrogen, fluoro and chloro;
R³ is selected from the group consisting of optionally substituted C₂-C₆ alkyl, optionally substituted aryl, optionally substituted heteroaryl and -NR⁴R⁵;
R⁴ and R⁵ are independently hydrogen or optionally substituted C₁ - C₆ alkyl;
or R⁴ and R⁵ combine with nitrogen to which they are attached to form optionally substituted 5 - 7 membered heterocycloalkyl;
wherein optionally substituted C₁-C₆ alkyl as R¹ or C₂-C₆ alkyl as R³ are C₁-C₆ alkyl or C₂-C₆ alkyl, respectively, optionally substituted with one or more substituents selected from the group consisting of halogen like F, OH, NH₂, NO₂, CN, C(O)OH, C(S)OH, C(O)NH₂, C(S)NH₂, S(O)₂NH₂, NHC(O)NH₂, NHC(S)NH₂, NHS(O)₂NH₂, C(NH)NH₂;
wherein optionally substituted C₂-C₆ alkenyl as R¹ is C₂-C₆ alkynyl optionally substituted with one or more substituents selected from the group consisting of halogen like F, OH, NH₂, NO₂, CN, C(O)OH, C(S)OH, C(O)NH₂, C(S)NH₂, S(O)₂NH₂, NHC(O)NH₂, NHC(S)NH₂, NHS(O)₂NH₂, C(NH)NH₂;
wherein optionally substituted C₂-C₆ alkynyl as R¹ is C₂-C₆ alkenyl optionally substituted with or more substituents selected from the group consisting of halogen like F, OH, NH₂, NO₂, CN, C(O)OH, C(S)OH, C(O)NH₂, C(S)NH₂, S(O)₂NH₂, NHC(O)NH₂, NHC(S)NH₂, NHS(O)₂NH₂, C(NH)NH₂;
wherein optionally substituted cycloalkyl, optionally substituted heterocycloalkyl, optionally substituted aryl or optionally substituted heteroaryl as R¹ or R³ or optionally substituted 5 - 7 membered heterocyloalkyl as R⁴ and R⁵ combined with the nitrogen to which they are attached are cycloalkyl, heterocycloalkyl, aryl, and heteroaryl , respectively each of which is optionally substituted with one or more substituents selected from the group consisting of halogen, OH, NH₂, NO₂, CN, C(O)OH, C(S)OH, C(O)NH₂, C(S)NH₂, S(O)₂NH₂, NHC(O)NH₂, NHC(S)NH₂, NHS(O)₂NH₂, C(NH)NH₂;
at each occurrence, alkenyl, by itself or as part of another substituent, is a straight or branched chain hydrocarbon having at least one carbon-to-carbon double bond;
at each occurrence, alkynyl, by itself or as part of another substituent, is a straight or branched chain hydrocarbon having at least one carbon-to-carbon triple bond;
at each occurrence, cycloalkyl, by itself or as part of another substituent, is a saturated or unsaturated, non-aromatic monocyclic, bicyclic or tricyclic carbon ring system of 3-10 ring members per ring; and
at each occurrence, heterocycloalkyl, by itself or as part of another substituent, is a saturated or unsaturated non-aromatic group having from 5 to 10 atoms in which from 1 to 3 carbon atoms in the ring are replaced by heteroatoms of O, S or N, and are optionally fused with benzo or heteroaryl of 5-6 ring members;
ii) a compound of Formula II 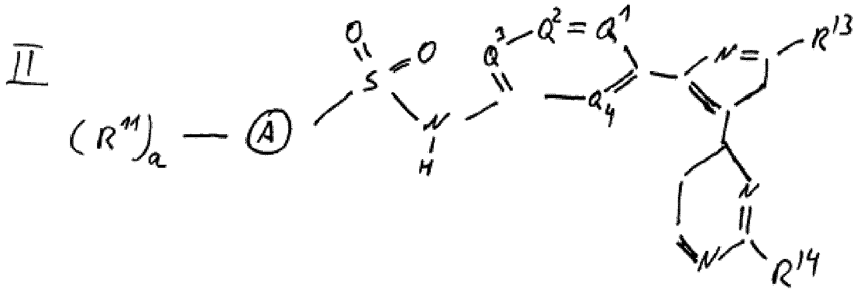 or a pharmaceutical acceptable salt, hydrate or solid thereof,
wherein a is 0,1, 2 or 3; each R¹¹ is the same or different and is independent is selected from halo, alkyl, haloalkyl, -OR¹⁶, -CO₂R¹⁶, -NR¹⁶R¹⁷ and -CN;
ring A is selected from C₃-C₆ cycloalkyl, phenyl 5-6 membered heterocycle and 5 - 6 membered heteroaryl, said heterocycle and said heteroaryl each having 1 or 2 heteroatoms selected from N, O and S each of Q¹, Q², Q³ and Q⁴ is CH, C-R¹² or N, wherein not more than one of Q¹, Q², Q³ and Q⁴ is N; each R¹² is the same or different is independently selected from halo, alkyl, haloalkyl, and - OR¹⁶; W is selected from -O- and -S-;
R¹³ is selected from H, alkyl, haloalkyl-alkylene-OH and -NR⁶R⁷, C₃₋₆ cycloalkyl, alkylene, -C(O)OH, -alkylene-NH₂, and Het;
wherein said R¹³C₃₋₆ cycloalkyl is optionally substituted with 1 or 2 substituents which are the same or different and are independently selected from halo, C₁₋₃ alkyl, haloC₁₋₃ alkyl, OH,OC₁₋₃ alkly, oxo, S(C₁₋₃ alkyl), SO₂, NH₂, N(H)C₁₋₃ alkly and N(C₁₋₃ alkyl)₂;
Het is a 5 - 6 membered heterocycle having 1 or 2 heteroatoms selected from N, O and S and optionally substituted with 1 or 2 substituents which are the same or different are each independently selected from halo, C₁₋₃ alkyl, haloC₁₋₃ alkyl, OC₁₋₃ alkyl, C₁₋₃ alkylene-O-C₁₋₃ alkyl, O-C₁₋₃ alkyl, OH, C₁₋₃ alkylene-OH, oxo, SO₂(C₁₋₃ alkyl), C₁₋₃ alkylene SO₂(C₁₋₃ alkyl), NH₂, N(H)C₁₋₃ alkyl, N(C₁₋₃ alkyl)₂, CN and CH₂CN;
R¹⁴ is selected from H, alkyl, haloalkyl, alkenyl-OR^{16,} R¹⁵-OR¹⁶, R¹⁵-CO₂, R¹⁶-, R¹⁵-SO₂R¹⁶, -R¹⁵-Het, -R¹⁵-C(O)-Het, N(H)R¹⁸, N(CH₃)R¹⁸, and -R¹⁵-NR¹⁶R¹⁷; each R¹⁵ is the same or different and is independently C₁ - C₄ alkylene, each R¹⁶ and each R¹⁷ is the same or different and is independently selected from H, alkyl, haloalkyl, -C(O) alkyl, and -C(O)-cycloalkyl;
R¹⁸ is selected from H, alkyl (optionally substituted by -OH), haloalkyl, C₃₋₆ cycloalkyl, R¹⁵C₃₋₆ cycloalkyl, Het², R¹⁵-Het², R¹⁵OR¹⁶, R¹⁵O-R¹⁵OR¹⁶, R¹⁵-C(OH)O₂R¹⁶,
-R¹⁵C(O)NR¹⁶R¹⁷, -R¹⁵-N(H)C(O)R¹⁶, -R⁵N(H)C(O)-R¹⁵-OR¹⁶, -R¹⁵N(H)C(O)₂-R16, -R¹⁵-NR¹⁶R¹⁷, -R¹⁵-S(O)₂R¹⁶, -R¹⁵-CN, and -R¹⁵-N(H)S(O)₂R¹⁶;
wherein said R¹⁸C₃₋₆ clycloalkyl is optionally substituted with 1 or 2 substituents which are the same or different and independently selected from halo, C₁₋₃ alkyl, haloC₁₋₃ alkyl, OH, OC₁₋₃ alkyl, oxo, S(C₁₋₃ alkyl), SO₂(C₁₋₃ alkyl), NH₂, N(H)C₁₋₃ alkyl and N(C₁₋₃ alkyl)₂, and N(H)SO₂C₁₋₃ alkyl; and
Het² is a 4-6 membered heterocycle having 1 or 2 heteroatoms selected from N, O and S and optionally substituted with 1, 2, 3, 4 or 5 C₁₋₃ alkyl or 1 or 2 substituents which are the same and different are independently selected from halo, C₁₋₃ alkyl, haloC₁₋₃alkyl, O-C₁₋₃alkyl, C₁₋₃alkylene-O-C₁₋₃alkyl, OH, C₁₋₃alkylene-OH, oxo, SO₂(C₁₋₃alkyl), C₁₋₃ alkylene-SO₂(C₁₋₃alkyl), NH₂, N(H)C₁₋₃alkyl, N(C₁₋₃alkyl)₂, N(H)SO₂C₁₋₃alkyl, C(O)(C₁₋₃alkyl), CO₂(C₁₋₃alkyl), CN, and -CH₂CN;
and R¹⁹ and R²⁰ are independently selected from H and alkyl.

4. The pharmaceutical composition according to claim 1 or 2 wherein the MEK-inhibitor is a compound of formula III or a pharmaceutically acceptable salt, hydrate or solid thereof 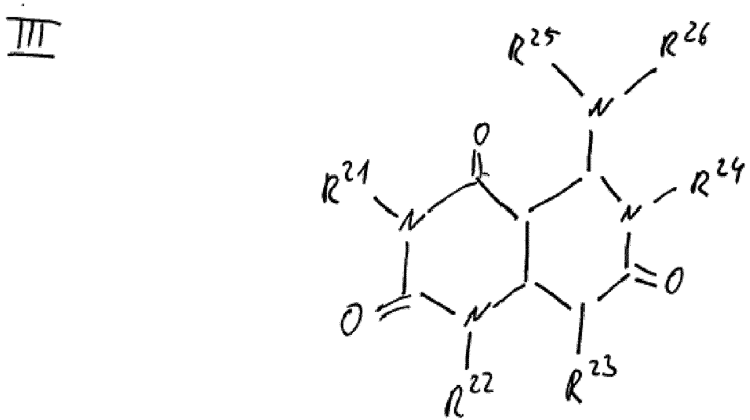
wherein R²¹, R²², and R²⁶ are the same or different and each is a C₁ - C₆ alkyl group, a C₂ - C₆ alkenyl group wherein the C₁₋₆ alkyl group and the C₂₋₆ alkenyl group are optionally substituted by 1 to 3 substituents selected from the group consisting of a halogen atom, a nitro group, a cyano group, a OR²⁷ wherein R²⁷ is a hydrogen atom or a C₁ - C₄ alkyl group, SR²⁷ wherein R²⁷ is a hydrogen atom or a C₁ - C₄ alkyl group, and NR²⁷R²⁸, -NR²⁷COR²⁸ wherein R²⁷ and R²⁸ are the same or different and each is a hydrogen atom or a C₁₋₄ alkyl group, COOR²⁷ wherein R²⁷ is a hydrogen atom or a C₁₋₄ alkyl group, in NR²⁷COR²⁸, R²⁸ is additionally a C₃ - C₁₂ carbon ring or a heterocylic group;
or - (CH₂)ₘ-Cy wherein
m is 0 or an integral of 1 to 4;
Cy is a ring of a C₃₋₁₂ carbon ring group or a heterocyclic group,
wherein the heterocyclic group is a saturated or unsaturated ring group having, besides carbon atom, 1 to 4 heteroatom selected from an O, N and S, the C₃-C12 carbon ring group and the heterocyclic group are optionally substituted by 1 to 5 substituents selected from the group consisting of a halogen atom, a nitro group, a cyano group, a C₁₋₈ alkyl group, a C₂₋₄ alkenyl group, a C₂₋₄ alkynyl group, OR²⁷, SR²⁷, NR²⁷R²⁸-NR²⁷COR²⁸ wherein R²⁷ and R²⁸ are defined as above, in NR²⁷R²⁸ and in NR²⁷COR²⁸, R²⁸ is additionally a C₃ - C₁₂ carbon ring or a heterocylic group;
R²³, R²⁴ and R²⁵ are the same or different and each is a hydrogen atom, a hydroxylgroup, a C₁₋₆ alkyl group, a C₂₋₆ alkenyl group, wherein the C₁₋₆ alkyl group and the C₂₋₆ alkenyl group are optionally substituted by 1 to 3 substituents selected from the group consisting of a halogen atom, a nitro group, a cyano group, a OR²⁷ wherein R²⁷ is a hydrogen atom or a C₁ -C₄ alkyl group, SR²⁷ wherein R²⁷ is a hydrogen atom or a C₁ - C₄ alkyl group, and NR²⁷R²⁸ wherein R²⁷ and R²⁸ are the same or different and each is a hydrogen atom or a C₁₋₄ alkyl group, COOR²⁷ wherein R²⁷ is a hydrogen atom or a C₁₋₄ alkyl group; or a combination of at least two of them forming a C₁ - C₄ alkylene group.

5. The pharmaceutical composition according to any one of the preceding claims wherein a combination of at least two BRAF inhibitors or a combination of at least one of a BRAF inhibitor and at least one of a MEK inhibitor is present.

6. The pharmaceutical composition according to claim 5 wherein a first BRAF inhibitor is a BRAF inhibitor of i) as defined above and a second BRAF inhibitor is a BRAF inhibitor of ii) as defined above or the at least one MEK inhibitor is a MEK inhibitor as defined in claim 4 and the at least one BRAF inhibitor is a BRAF inhibitor i) and/or ii) as defined in claim 3.

7. The pharmaceutical composition according to claim 1 or 2 wherein the BRAF inhibitor is ARQ 736, ASN003, BGB-283, CEP-32496, CCT3833, Dabrafenib, Encorafenib, GDC-0879, HM95573, LXH254, LY3009120, PLX3603, PLX4720, PLX8394, RAF265, Regorafenib, RO5126766, Sorafenib, TAK-580, Vemurafenib, XL281 and/or the MEK inhibitor is ARRY-300, AS703988, AZD8330, BI 847325, Binimetinib, Capmatinib, Cobimetinib, Crizotinib, E6201, GDC-0623, PD0325901, Pimasertib, Refametinib, RO4987655, Selumetinib, Trametinib, TAK-733, WX-554, and/or the ERK inhibitor is BVD-523, CC-90003, GDC-0994, KO-947, Lapatinib, LTT462, LY 3214996 or ONC201.

8. A pharmaceutical composition according to any one of the preceding claims
wherein the BRAF inhibitor of i) is Vemurafenib and/or the BRAF inhibitor of ii) is Dabrafenib and/or the MEK inhibitor of formula III is Trametinib.

9. A pharmaceutical composition according to any one of the preceding claims comprising beta-Sitosterol and Vemurafenib.

10. The pharmaceutical composition according to any one of the preceding claims for use in the treatment of cancer, including Low grade glioma, Pediatric glioma, High grade glioma (pleomorphic xantoastrocytoma), Thyroid carcinoma (medullary, papillary, anaplastic), Oesophageal carcinoma, Meningioma, Soft tissue sarcoma, Lymphoblastic leukaemia, Hairy cell leukaemia, Non Hodgkin lymphoma, Langerhans cell histiocytosis, Multiple myeloma, Ependymoma, ATRT (atypical teratoid rhabdoid tumour), Craniopharyngioma, Neuroblastoma, Non-small cell lung, cancer (NCSLC), Malignant pleural mesothelioma, Thymic carcinoma, NET (neuroendocrine tumour), NEC (neuroendocrine carcinoma), Pheochromocytoma, Paraganglioma, Melanoma, Colorectal cancer, Seminoma, Non-seminoma, Gastrointestinal stromatumor, Gastric carcinoma, Duodenal neoplasm of gastric phenotype (DNGP), Pancreatic cancer, Small intestinal adenocarcinoma, Wilms tumour (Nephroblastoma), Renal cell carcinoma, Urothelial carcinoma, Bladder cancer (transitional cell carcinoma of the bladder), Prostate cancer, Malignant phyllodes tumor of the breasts, Biliary tract cancer, Ovarian cancer, Breast cancer (metastatic), Malignant peripheral nerve sheath tumor (MPNST), Vulvar cancer, Cutaneous squamous cell carcinoma, Squamous cell anal carcinoma, Histiocytic sarcoma, Hepatocellular carcinoma, Metanephric stromal tumour (benign), Renal cell carcinoma, Upper tract urothelial carcinoma, Gastric endocrine carcinoma (carcinoid), Adrenocortical carcinoma, Ewing sarcoma, Embryonal rhabdomyosarcoma, and Malignant phyllodes tumour.

11. The pharmaceutical composition for use in treating cancer according to claim 10 wherein the cancer is a metastatic cancer, in particular, metastatic melanoma.

12. A method of treating an individual suffering from cancer comprising the step of administering to said individual a pharmaceutical composition according to any one of claims 1 to 9 whereby beta-Sitosterol or a homolog thereof and the at least one of a BRAF-inhibitor and a MEK-inhibitor are administered simultaneously, separately or sequentially.

13. The method of treating an individual suffering from cancer according to claim 12 wherein said method is a method of treating an individual suffering from metastatic cancer, in particular, metastic melanoma.

14. The method for treating an individual suffering from cancer according to claim 11 or 12 wherein administration is orally or intravenously.

15. The pharmaceutical composition according to any one of claims 1 to 9 further comprising a pharmaceutically acceptable carrier, diluent or excipient.
